# EUROPEAN PATENT APPLICATION

(11) **EP 4 328 855 A1**
(43) Date of publication of application: **28.02.2024**
(21) Application number: 22191664.6
(22) Date of filing: 23.08.2022
(51) Int. Cl.: G06T 7/00

(54) **METHODS AND SYSTEMS FOR IDENTIFYING A CANDIDATE MEDICAL FINDING IN A MEDICAL IMAGE AND PROVIDING THE CANDIDATE MEDICAL FINDING**

(71) Applicant: Siemens Healthineers AG, 91301 Forchheim (DE)
(72) Inventor: Ritter, Juliane, 91096 Möhrendorf (DE)
(74) Representative: Siemens Healthineers Patent Attorneys

(57) **Abstract**

Provided are computer-implemented methods and corresponding systems for providing a candidate medical finding based on the analysis of a medical image (IM). The methods and systems are based on the following steps:
- obtaining (S10) the medical image (IM) depicting a body part of a patient,
- generating (S20) a first set of candidate medical findings (CMF1) by subjecting the medical image (IM) to a first medical findings detection process (D-PROC1),
- generating (S30) a second set of candidate medical findings (CMF2) by subjecting the medical image (IM) to a second medical findings detection process (D-PROC2) different than the first medical finding detection process (D-PROC1),
- obtaining (S40) a region of interest (ROI) in the medical image (IM),
- identifying (S50), in the region of interest (ROI), at least one candidate medical finding (ADD-CMF) comprised in the second set of candidate medical findings (CMF2) and not comprised in the first set of candidate medical findings (CMF1), and
- providing (S50) the at least one candidate medical finding (ADD-CMF).

## Description

The present invention relates to methods and systems for identifying medical findings comprised in a medical image. Further, the present invention relates to methods and systems for providing such identified medical findings.

Advances in medical imaging, e.g., employing computed tomography or magnetic resonance systems, allow for reproducing tiniest changes in the anatomy of a patient. As a result of the increased performance of these systems, there is increased focus on the early detection of diseases with improved chances of success of the following treatment. However, for radiologists, this increased focus also has negative aspects. The procedure of visually analyzing radiology images is often challenging. For instance, the density and tissue type of organs are highly varied and in turn present a high variety of visual features. Additionally, background visual patterns can obscure the early signs of malignancies which may then be easily overlooked by the human eye. Therefore, the manual classification of the spatial distribution of abnormalities or patterns inevitably leads to errors owing to mistakes, human error, and/or details too fine for the human eye to detect. Thus, the analysis of medical images may lead to false negatives which may cause missed treatment. Likewise, the evaluation may prompt false positives which may cause unwanted psychological and sub-optimal downstream diagnostic and treatment consequences. What is more, the reliable detection of abnormalities and/or features in medical images often requires highly experienced physicians further increasing their workload. Moreover, the human component in evaluating image data adds a degree of subjectivity which is often unwanted.

To cope with such problems, computer- aided detection (CADe) and computer-aided diagnosis (CADx) systems are being developed. Hereafter both types of systems will be referred to as CAD systems. CAD systems are technologies to help radiologists interpret medical images. A common use of CAD systems is to automatically identify suspicious regions in a medical image. Such suspicious regions may contain image patterns indicative of abnormalities which may comprise cancerous growths, masses, abscesses, lacerations, calcifications, lesions and/or other irregularities within biological tissue and which can cause serious medical problems if left undetected.

Basically, an ideal CAD system should be able to securely identify all actual abnormalities without generating any false positives. This may sound straightforward but is very difficult to achieve in practice as this means fulfilling two conflicting requirements. At the one hand, CAD systems have to be highly sensible so that no potentially relevant objects remain undetected. On the other hand, a highly sensitive detection inevitably increases the likelihood of generating false positives. In other words, such a (over-)sensitive detection loses its specificity.

As one compromise, it is state-of-the art to set-up CAD systems with an operating point, that balances sensitivity and specificity of the detection to create the most useful output in most of the cases from a statistical point of view (balancing out the likelihood of false positives and false negatives). Now, whilst e.g., a balanced operating point is a good solution from a statistical point of view, it still is a trade-off decision. By consequence, there is the remaining statistical probability that CAD systems miss out on detecting true positive lesions. Thus, users may feel the need to manually add lesions - which is not only cumbersome but potentially degrades the user's trust in the CAD system.

It is an object of the present invention to address this conflict and provide a method and a corresponding system capable of securely identifying and providing medical findings (i.e., objects of pathological relevance) in medical images. In particular, it is an object of the present invention to provide an improved computer-implemented method for providing medical findings and supporting a user/physician/radiologist/pathologist in deriving a medical diagnosis from a medical image.

In the following, the technical solution according to the present invention is described with respect to the claimed systems as well as with respect to the claimed methods. Features, advantages or alternative embodiments described herein can likewise be assigned to other claimed objects and vice versa. In other words, claims addressing the inventive method can be improved by features described or claimed with respect to the systems. In this case, e.g., functional features of the methods are embodied by objective units or elements of the systems.

According to an aspect, a computer-implemented method for providing a candidate medical finding comprised in a medical image is provided. The method comprises a plurality of steps. A first step is directed to obtain the medical image depicting a body part of a patient. A further step is directed to generate a first set of candidate medical findings by subjecting the medical image to a first medical findings detection process. A further step is directed to generate a second set of candidate medical findings by subjecting the medical image to a second medical findings detection process different than the first medical finding detection process. A further step is directed to obtain a region of interest in the medical image. A further step is directed to identify, for (or in or within) the region of interest, at least one candidate medical finding comprised in the second set of candidate medical findings and not comprised in the first set of candidate medical findings. A further step is directed to provide the at least one candidate medical finding.

In particular, the medical image may be a two-dimensional image. In particular, the medical image may be a three-dimensional image. In particular, the medical image may be a four-dimensional image, where there are three spatial and one time-like dimensions. Further, the medical image may relate to a medical image data set comprising a plurality of medical images.

The medical image may depict a body part of a patient in the sense that it contains two or three-dimensional image data of the patient's body part. The medical image may be representative of an image volume or a cross-section through the image volume. The patient's body part may be comprised in the image volume.

The medical image comprises image data, for example, in the form of a two- or three-dimensional array of pixels or voxels. Such arrays of pixels or voxels may be representative of intensity, absorption or other parameters as a function of three-dimensional position, and may, for example, be obtained by suitable processing of measurement signals obtained by a medical imaging modality.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of "hard" X-rays produced and detected by a spatially rotating instrument. The resulting attenuation data (also referred to as raw data) is processed by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

Accordingly, the depicted body part of the patient in general will comprise a plurality of anatomies and/or organs. Taking a chest image as an example, the medical image may show lung tissue, the rib cage, lymph nodes and others.

A medical image data set may comprise a plurality of images or image slices. The slices respectively show a crosssectional view of the image volume. The slices may comprise a two-dimensional array of pixels or voxels as image data. The arrangement of slices in the medical image data set may be determined by the imaging modality or by any post-processing scheme used. Further, slices may artificially be defined in the imaging volume spanned by the medical image data set. Optionally, this may happen as a function of the image data comprised in the medical image data set in order to optimally pre-process the medical image data set for the ensuing diagnostic workflow.

The medical image may be stored in a standard image format such as the Digital Imaging and Communications in Medicine (DICOM) format and in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

"Obtaining" may mean that the medical image data set is directly obtained from the medical imaging modalities. Further "obtaining" may mean that the medical image data set is retrieved from an appropriate memory such as a picture archiving and communication system (PACS) or any other suitable medical image storing facility.

First and second sets of candidate medical findings respectively may comprise one or more individual candidate medical findings. Each candidate medical finding may pertain to corresponding image data in the medical image. A candidate medical finding may indicate a certain condition or pathology of the patient with a certain likelihood. The condition or pathology may be relevant for the diagnosis of the patient.

A candidate medical finding may relate to an anatomical structure that differentiates the patient from other patients. Candidate medical findings may be located within different organs of the patient (e.g., within the lung of a patient, or within the liver of a patient) or in between the organs of the patient. In particular, a candidate medical finding may also relate to a foreign body.

In particular, a candidate medical finding may relate to a neoplasm (also denoted as "tumor"), in particular, a benign neoplasm, an in-situ neoplasm, a malignant neoplasm and/or a neoplasm of uncertain/unknown behavior. In particular, a candidate medical finding may relate to a nodule, in particular, a lung nodule. In particular, a candidate medical finding may relate to a lesion, in particular, a lung lesion.

A candidate medical finding in general may indicate a potential finding for further review either by a user or a subsequent computer-implemented processes such as a classification process for identifying "real" medical findings within the candidate medical findings. As such, the candidate medical findings may also comprise "false positives" that do not turn out to relate to real medical findings.

The first detection process may comprise inputting the medical image into a first automated detection algorithm configured to detect candidate medical findings in medical images. Likewise, the second detection process may comprise inputting the medical image into a second automated detection algorithm configured to detect candidate medical findings in medical images. The second detection algorithm may be different than the first detection algorithm. According to other examples, the second detection process may comprise inputting the medical image into the first detection algorithm which is adjusted differently than in the first detection process, e.g., by using different parameters.

Using different detection processes may generally mean that the first set of candidate medical findings is different than the second set of medical findings.

In particular, the second set of candidate medical findings may comprise different and/or additional candidate medical findings as compared to the first set of candidate medical findings. In other words, the second set of candidate medical findings may comprise at least one candidate medical finding not comprised in the first set of candidate medical findings. Further, the second set of candidate medical findings may comprise one or more candidate medical findings also comprised in the first set of candidate medical findings. According to some examples, the second set of candidate medical findings may comprise more candidate medical findings than the first set of candidate medical findings. According to other examples, the second set of candidate medical findings may comprise fewer candidate medical findings than the first set of candidate medical findings. According to some examples, the first set of candidate medial findings may be a subset of the second set of candidate medical findings.

A region of interest is to be understood as a group of image elements like pixels or voxels within the medical image. The region of interest comprises at least one, but preferably numerous image elements of the medical image. The region of interest may represent an area within the medical image, which is of specific interest, e.g., for the user analyzing the medical image. A region of interest may generally relate to a part or to a plurality of different parts of the medical image. The region of interest may have an arbitrary shape, preferably the region of interest is of circular or quadratic form. Further, the region of interest may comprise a plurality of individual sub-regions.

According to some examples, the region of interest does not comprise any candidate medical findings of the first set of candidate medical findings. According to some examples, the region of interest does not comprise any candidate medical findings of the first set of candidate medical findings but at least one candidate medical finding of the second set of candidate medical findings.

The region of interest may be defined by a user or semi-automatically or automatically by the computer-implemented method.

Thus, obtaining the region of interest may be based on processing one or more user inputs to designate the region of interest in the medical image. For instance, such user inputs may comprise clicking in the medical image and defining a region of interest on that basis.

Further, obtaining the region of interest may comprise automatically detecting the region of interest, e.g., based on supplementary data associated with the medical image. For instance, such supplementary data may comprise information about particularly relevant regions within the medical image. E.g., the relevance of these regions may be due to the fact that they already played a role in earlier examinations of the patient which, in turn, may be encoded in the aforementioned supplementary data.

In the step of identifying, at least one candidate medical finding located in or at least in proximity to the region of interest is identified from the second set of candidate medical findings. Since the at least one candidate medical finding is not comprised in the first set of candidate medical findings, it may be conceived as an additional candidate medical finding with respect to the first set of candidate medical findings - which has been specifically identified for the region of interest. Identifying may mean that the second set of candidate medical findings is searched for candidate medical findings that are located within or in proximity to the region of interest. Further, identifying may comprise checking whether or not these candidate findings are already comprised in the first set of candidate medical findings.

The at least one candidate medical finding may be provided by outputting the at least one candidate medical finding. This may comprise outputting the at least one candidate medical finding to a user via a user interface, in particular, by displaying the at least one candidate medical finding in a graphical user interface. Further, providing may comprise storing the at least one candidate medical finding in an appropriate data format, e.g., together with the first set of candidate medical findings. Further, providing may comprise including the at least one candidate medical finding in a output data structure or generating a output data structure based on the at least one candidate medical finding. According to some examples, the output data structure may be an electronical medical report.

According to some examples, providing the at least one candidate medical finding may also comprise an audio output to a user via a user interface. In particular, the audio output may indicate the at least one candidate medical finding. In particular, the audio output may comprise a sound indicating the presence of the at least one candidate medical finding. In particular, the audio output may comprise a speech output to a user. In particular, the speech output provides details of the candidate medical finding. Thereby, audio outputs may provide the user with additional support in the reading and reporting workflow.

By using two different detection processes, complementary sets of candidate medical findings can be generated. The first set of candidate medical findings can be considered as a "standard" set generated with a detection process which usually works well. The second set of candidate medical findings can then be seen as providing alternative candidate medical findings which, in some cases, may provide additional insights for a user but in other case may comprise outliers which are not relevant for a user. To alleviate this, candidate findings from the second set of candidate medical findings are not considered in an arbitrary but in a specific manner, namely in connection with a specific region of interest. This offers a workflow enabling to selectively include individual candidate medical findings of the second set for a region of interest without having to consider the rest of the second set. This enables identifying and providing medical findings in medical images in a more secure and complete manner without increasing the burden for a user to review an increased number of false positives. With that, the user is better supported to derive a medical diagnosis from a medical image.

According to an aspect, a computer-implemented method for providing a candidate medical finding from a medical image is provided. The method comprises a plurality of steps. A first step is directed to obtain the medical image depicting a body part of a patient. A further step is directed to generate a first set of candidate medical findings by subjecting the medical image to a first medical findings detection process. A further step is directed to generate a second set of candidate medical findings by subjecting the medical image to a second medical findings detection process different than the first medical findings detection process. A further step is directed to obtain a region of interest in the medical image.

A further step is directed to determine if the region of interest comprises at least one candidate medical finding comprised in the second and not comprised in the first set of candidate medical findings. A further step is directed to provide the at least one candidate medical finding, if the region of interest comprises at least one candidate medical finding comprised in the second and not comprised in the first set of candidate medical findings.

According to an aspect, the step of generating the first set of candidate medical findings comprises detecting candidate medical findings in the medical image with a first sensitivity level, and the step of generating the second set of candidate medical findings comprises detecting candidate medical findings in the medical image with a second sensitivity level higher than the first sensitivity level.

The terms "level of sensitivity" or "sensitivity level" as used herein may pertain to the sensitivity with which image data is identified as suspicious and thus pertaining to a candidate medical finding. In general, the higher the level of sensitivity is, the higher is the likelihood that a certain feature present in the medical image is identified as a candidate medical finding. Thereby, the level of sensitivity may pertain to a decision threshold above which image data is qualified as a candidate medical finding. In general, higher levels of sensitivity in the medical findings detection process will produce more candidate medical findings. Hence, higher levels of sensitivity will allow to capture more (and ideally all) actual medical findings in the medical image but will also lead to more false positives, i.e., candidate medical findings that do not relate to actual medical findings. In other words, a higher level of sensitivity may entail a reduced level of specificity.

The first level of sensitivity may be adjusted such that it constitutes a reasonable compromise between sensitivity and specificity for the medical image. Compared to that, the second level of sensitivity deliberately accepts limitations in specificity to allow for a more sensible sampling of the medical image for candidate medical findings. Thus, the image analysis with the second level of sensitivity (which is higher than the first level of sensitivity) may thus be seen as a complementary analysis step designed to find additional candidate medical findings - in addition to the candidate medical findings found at the first level of sensitivity.

Generally, the second level of sensitivity may be comparatively high. Thus, the second level of sensitivity may not be suited, in all scenarios, to provide a set of medical findings with reasonably low numbers of false positives that can readily be separated out in a next step.

According to some examples, the sensitivity and the corresponding specificity of the detection processes behave reciprocally. The higher the sensitivity, the lower the specificity and, thus, the higher the number of false positives.

According to some examples, the step of generating the first set of candidate medical findings comprises detecting candidate medical findings in the medical image with a first specificity level, and the step of generating the second set of candidate medical findings comprises detecting candidate medical findings in the medical image with a second specificity level lower than the first specificity level.

According to some examples, the first level of sensitivity may be between 70% to 90% meaning that 70% to 90% of all "real" medical findings are detected as candidate medical findings in the first detection process. In turn, the specificity may be as high as 95% or higher in the first detection process such that the number of false positives is rather low. By contrast, the sensitivity of the second detection process may be 90% or higher so that most or all of the "real" medical findings will be detected. In turn, the specificity of the second detection process may be lower than 90% so that also the number of false positives may be higher.

By analyzing the medical image at two different sensitivity levels, a second sensitivity level may be held available as a backup for image regions where the first sensitivity level does not occur to be sufficient. The second sensitivity level generally may be too sensitive (that is, too unspecific) if the entire image is considered but can provide additional insights for specific regions such as the region of interest on demand. Accordingly, the conflicting requirements of having a close to complete detection of all findings and a minimal number of false positives can be addressed.

According to an aspect, in the step of generating the first set of candidate medical findings, the first medical findings detection process comprises applying a first medical findings detection algorithm to the medical image, the first medical findings detection algorithm operating at the first sensitivity level, and in the step of generating the second set of candidate medical findings, the second medical findings detection process comprises applying a second medical findings detection algorithm to the medical image, the second medical findings detection algorithm operating at the second sensitivity level.

The medical findings detection algorithms may generally be configured to detect candidate medical findings in medical images. For instance, the findings detection algorithms may have two stages: the detection stage for detecting potentially relevant patterns in image data and the classification stage one for classifying the potentially relevant patterns either as candidate medical findings or as false positives to be discarded. In principle, a plethora of functionalities and methods is known for such computer aided detection and classification of candidate medical findings - all of which may be implemented in the findings detection algorithms. For instance, reference is made to US 2009 / 0 092 300 A1,

US 2009 / 0 067 693 A1, and US 2016 / 0 321 427 A1, the contents of which are incorporated herein in their entirety by reference. In particular, findings detection algorithms may comprise one or more machine learned functions trained to detect and classify candidate medical findings if applied to a medical image. Suitable trained functions for this task include (artificial) neural networks, such as convolutional neural networks.

According to some examples, the medical findings detection algorithms may be configured such that their level of sensitivity (and in turn their specificity) can be adjusted. According to some examples, the sensitivity and the specificity of the medical findings detection algorithms may be adjusted with the point of operation of the respective medical findings detection algorithm.

The usage of medical findings detection algorithms allows for an efficient and reproducible detection of candidate medical findings. Since the algorithms are operating at two sensitivity levels, complementary results can be generated that can be selectively combined.

According to an aspect, the second medical findings detection algorithm and the first medical image algorithm are the same.

According to some examples, the first and second detection algorithms are the same but are working with different points of operation in the first and second detection process. According to some examples, the point of operation for the second detection process is the most sensitive point of operation available for the detection algorithm.

Using the same medical findings detection algorithm for the first and second detection process - albeit with different points of operation - allows for an efficient generation of the different sets of candidate medical findings and at the same time ensures that the candidate medical findings are readily comparable.

According to an aspect, the method further comprises the step of setting the first sensitivity level based on any one of:
- an input of the user directed to setting the second sensitivity level,
- the medical image, and/or
- supplementary non-image data associated with the medical image.

The input of the user may for instance be input via a corresponding user interface. With that, a user can set his individual preferences regarding the level of sensitivity. According to some examples, the user input may be directed to set the point of operation of the medical findings detection algorithm in the first detection process.

As an alternative, setting the first sensitivity level may be based on the medical image. With that, a sensitivity level may be determined which is adapted to the image data comprised in the medical image. To set the first sensitivity level, the medical image may be analyzed for characteristics influencing the detection process.

In addition to that or as an alternative, the first level of sensitivity may be set based on supplementary non-image data associated with the medical image. Such data may indicate the diagnostic task to be performed based on the medical image, properties of the medical image, and/or previous settings used upon analyzing medical images of the same patient or similar patients. With that, the first sensitivity level can be optimally adapted to the diagnostics to be performed with the medical findings detection process.

According to an aspect, the method further comprises the step of setting the second sensitivity level based on the first sensitivity level.

By (automatically) setting the second sensitivity level based on the first sensitivity level, it can be ensured that the second sensitivity level optimally complements the first detection process.

Alternatively, the second sensitivity level may be set based on any one of:
- an input of the user directed to setting the second sensitivity level,
- the medical image, and/or
- supplementary non-image data associated with the medical image.

According to an aspect, the first and the second medical findings detection process are run in parallel. In particular, the first and second medical findings detection algorithms may process the medical image in parallel.

With the parallel processing, it can be ensured that there are no waiting times for the user and the detection results generated in the first and second detection process are simultaneously available.

According to an aspect, the step of identifying the at least one candidate medical finding comprises determining if the region of interest comprises the at least one medical finding, wherein the step of providing the at least one medical finding is based on the step of determining.

In particular, the at least one candidate medical finding may be provided if the region of interest does comprise the at least one candidate medical finding.

By checking if the region of interest comprises candidate medical findings of the second set, it can be ensured that the candidate medical findings are selectively pulled from the second set of candidate medical findings. Accordingly, additional results can selectively be provided for the region of interest without sacrificing the overall specificity of the method.

According to an aspect, the method further comprises providing a notification (e.g., to a user via a user interface) indicating that the region of interest does not comprise any candidate medical finding from the second set of candidate medical findings, based on the step of determining if the region of interest comprises the at least one candidate medical finding.

Accordingly, a user may be notified if the region of interest does not contain additional medical findings from the second set. Accordingly, the user can get a confirmation that a region of interest she or he considered suspicious does not comprise further medical findings. The user may then focus on other candidate medical findings and/or define alternative regions of interest.

According to an aspect, the step of obtaining the region of interest comprises:
- generating a representation of the medical image for displaying to a user in a user interface, wherein, optionally, the representation comprises one or more indications of the candidate medical findings comprised in the first set of candidate medical findings,
- providing the representation to the user in the user interface,
- receiving, optionally via the user interface, a user input directed to indicate the region of interest in the representation, and
- defining the region of interest in the medical image based on the user input directed to indicate the region of interest.

The representation may be a two-dimensional representation image rendered from the medical image for displaying to a user in a user interface. The representation may comprise a plurality of image pixels. In particular, the representation may be a two-dimensional rendering of the medical image. Two-dimensional renderings may, in general, rely on known rendering procedures, such as ray-casting, ray-tracing, texturerendering or the like. According to some examples, the rendering may be such that the region of interest and/or any candidate medical findings from the first set of candidate medical findings are displayed in conjunction with the image data of the medical image. Of note, according to some examples, the results based on the second detection process are not shown to the user in the representation at first per default as only the results from the first detection process shall be automatically integrated and displayed in the representation.

An indication in this context man mean that the underlying candidate medical findings are highlighted in the representation. This may mean that the candidate medical findings are visually enhanced in brightness, color, and/or intensity. In addition to that or as an alternative, the candidate medical findings may be highlighted using symbols. Further, candidate medical findings may be highlighted using labels comprising semantic expressions. The highlighting or labeling may be carried out based on information from the detection processes, such as position, extension or volume of the respective candidate medical finding. Further, the indication(s) may be provided in the form of an image overlay which can be shown or hidden.

The user input may be any input directed to designate a region of interest. The user input may comprise a voice command or any other, in particular, physical input into a user interface, in particular, a graphical user interface. For instance, the user may use input devices like a computer-mouse, a trackball device, a smart pen, a touch pad, a touch sensitive display, etc. Further, the user input may be captured by eye tracking or by tracking gestures. The user input may, in particular, comprise designating a region of interest directly in the representation, e.g., by clicking or drawing contours in a specific location in the representation. The user input may be such that it fully lines out the region of interest or such that it indicates only parts or even only a point in the representation which is then automatically completed to derive the region of interest.

By providing a two-dimensional representation image for visual inspection and by allowing to custom-define the region of interest, the method is specifically adapted to interact with a user. This enables the method to be adapted to the medical image under consideration, the current pattern of interest, and the needs of the user. With that, the user can individually designate image region where she or he suspects additional candidate medical findings in addition to the candidate medical findings of the first set which are already shown. Thus, additional findings can selectively be pulled on demand.

According to an aspect, the step of defining the region of interest is associated to a tool activable by the user via the user interface and defining the region of interest based on the user input is only possible after the tool has been activated by the user. With that, it can be ensured that the process of pulling additional candidate medical findings from the second set is only carried out if desired and not for any unspecific interaction of the user with the representation such as zooming or panning.

According to an aspect, the user input is directed to a location in the representation and the region of interest is defined as a region around the location within a preset distance from the location.

In other words, a user input may identify a certain point or pixel within the representation which is then extended to a region of interest of a larger more reasonable size for pulling further candidate medical findings from the second set.

The preset distance can be conceived as an offset in order to create some error tolerance of the system. Thus, if a user input is such that a nearby candidate second medical finding would be missed, such findings may still be considered if the user input is extended by the offset to define the region of interest. The predetermined distance may, for instance, be measured in pixels of the representation.

According to an aspect, the method further comprises receiving, optionally, via the user interface, a user input directed to set the predetermined distance, and presetting the distance based on the user input.

With that, a user can individually adjust the dimensions of the region of interest that will be defined based on a subsequent user input directed to define the region of interest. If the user considers an entire region as relevant, she or he can set the distance to greater values as compared to a case where more local feedback is desired.

According to an aspect, the user input is directed to a location in the representation and the region of interest is defined based on an image property of image data of the medical image relative to the location.

According to some examples, the image property may comprise at least one of: an intensity, in particular a mean intensity, a color value, in particular a mean color value, an intensity window, and/or an association to an organ depicted in the medical image. Thereby, the association to an organ may be determined based on one or more organ segmentations.

According to some examples, the step of defining the region of interest comprises extracting the image property from image data relative of the location.

According to some examples, defining the region of interest comprises assigning portions of the medical image to the region of interest based on the image property.

According to some examples, assigning comprises assigning those image portions of the medical image to the region of interest which have a corresponding image property matching the image property of image data relative to the location, optionally, within a preset tolerance margin or range. Thereby, the tolerance margin may correspond to an offset, threshold, or corridor.

According to some examples, the step of defining the region of interest comprises extracting the corresponding image properties from the image portions.

According to some examples, the method further comprises receiving, optionally, via the user interface, a user input directed to set the preset tolerance margin and presetting the tolerance margin based on the user input.

With the definition of the region of interest based on image properties, a user input can automatically be expanded to portions of the medical image which have similar image properties. Thereby, the portions may be connected or separated. In other words, an automated region growing process may be provided to capture further relevant portions of the medical image based on the user input.

Taking CT image data as an example, the medical image may be intensity filtered based on the user input. Each voxel of a CT image data set usually has an intensity value that represents the attenuation of X-rays at the corresponding position in the volume as determined from the CT measurements (commonly measured in Hounsfield or HU units). Due to this relation, a kind of segmentation can be performed based solely on a thresholding of the voxel intensity values. For example, all voxels having intensity values in a particular range or tolerance margin may be considered to represent bone. Accordingly, the candidate medical findings of the second set may be automatically provided for all bone-like structures depicted in the medical image.

According to an aspect, the step of providing comprises including an indication of the at least one candidate medical finding in the representation so as to generate an updated representation, further with the step of displaying the updated representation to the user in the user interface.

If the region of interest comprises additional candidate medical findings of the second set, these are specifically indicated in the representation alongside the (already displayed) candidate medical findings of the first set. Accordingly, the user is provided with a visual verification whether or not the region of interest does comprise additional candidate medical findings which were found in the second detection process only.

According to an aspect, the indication of the at least one candidate medial finding is visually different from the indication from the indication of the medical findings of the first set of candidate medical findings.

This has the benefit that a user may immediately tell the displayed medical findings apart depending on the detection process they were generated with. This provides additional insights for the user especially if she or he revisits the representation at a later point in time.

According to an aspect, the method further comprises indicating to the user, optionally via the user interface, if the region of interest does not comprise a candidate medical finding of the second set of candidate medical findings.

Accordingly, a user gets a confirmation that the region of interest does not contain additional medical findings from the second set making the procedure clearer to the user.

According to an aspect, each of the candidate medical findings of the second set of candidate medical findings is associated with a confidence value, and, in the step of identifying the at least one candidate medical finding, only such candidate medical findings of the second set of candidate medical findings are considered the confidence values of which are above a preset confidence threshold.

The confidence value may quantitively indicate a likelihood that a given candidate medical finding may relate to a "real" medical finding. In other words, the confidence value may provide an indication for the individual specificity of a candidate medical finding. The confidence value may be determined as a by-product in the second detection process and, e.g., output by the corresponding medical findings detection algorithm.

By evaluating individual confidence values upon identifying the at least one medical finding, an additional filter can be applied further ensuring that the user is only presented with likely candidates. Accordingly, the rate of false positives can be further decreased. The confidence threshold may be set automatically.

According to an aspect, the method further comprises receiving, optionally, via the user interface, a user input directed to set the confidence threshold, and presetting the confidence threshold based on the user input.

With that, a user is provided with yet another way to influence the processing and interactively control what is presented to her or him as the detection results.

According to an aspect, the step of providing comprises associating the at least one candidate medical finding to the first set of candidate medical findings, and, preferably, including the at least one candidate medical finding in the first set of candidate medical findings so as to generate a modified set of candidate medical findings for further use.

With that, the at least one candidate medical finding is "promoted" as a finding of the first set and can subsequently be accessed via the first set, e.g., during further processing or for reporting and documentation purposes.

According to an aspect, the method further comprises:
- providing an notification of the presence of the at least one candidate medical finding to a user via a user interface, preferably in the form of an indication in the representation, and
- receiving a user input directed to confirm or reject the at least one candidate medical finding based on the notification, wherein
- the step of providing is based on the user input directed to confirm or reject.

In particular, the at least one candidate medical finding may be associated with and/or included in the first set of candidate medical findings only if the user input was directed to confirm the at least one candidate medical finding.

Accordingly, the user is provided with additional means to control and influence the processing. In particular, the user is given another possibility to inspect the result of the processing and decide if the results are acceptable.

According to an aspect, a system for providing a candidate medical finding of a body part of a patient based on a medical image of the body part is provided. The system comprises an interface unit and a computing unit. The interface unit is configured to obtain the medical image. The computing unit is configured to generate a first set of candidate medical findings by subjecting the medical image to a first medical findings detection process. The computing unit is further configured to generate a second set of candidate medical findings by subjecting the medical image to a second medical findings detection process different than the first medical findings detection process. The computing unit is further configured to obtain a region of interest in the medical image. The computing unit is further configured to identify, in (or for or within) the region of interest, at least one candidate medical finding comprised in the second set of candidate medical findings and not comprised in the first set of candidate medical findings. The computing unit is further configured to provide the at least one candidate medical finding.

The computing unit may comprise a medical findings generation unit configured to generate candidate medical findings by processing medical images and, thereby, to execute the first and second medical findings detection processes. In particular, the medical findings generation unit may be configured to host, run and/or apply the medical findings detection algorithm. The computing unit may further comprise a user interaction unit configured to define a region of interest in the medical image based on a corresponding user input. Optionally, the user interaction unit may further be configured to generate a representation (or visualization) of the medical image for the user on the basis of which the user can input a user input directed to define a region of interest in the medical image and/or on the basis of which the at least one candidate medical finding may be provided. The computing unit may further comprise a medical findings identification unit configured to identify, in the region of interest, at least one candidate medical finding comprised in the second set of candidate medical findings and not comprised in the first set of candidate medical findings.

The computing unit may be realized as a data processing system or as a part of a data processing system. Such a data processing system can, for example, comprise a cloudcomputing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medial image. The interface unit may be further adapted to interface with one or more users of the system, e.g., by displaying the result of the processing by the computing unit to the user (e.g., in a graphical user interface) or by allowing the user to adjust parameters for image processing or visualization.

According to other aspects, the invention further relates to an image analysis system comprising the above system and a medical image system (or medical information system) configured to acquire, store and/or forward medical images. Thereby, the interface unit is configured to receive the medical image form the medical image system.

According to some examples, the medical image system comprises one or more archive stations for storing medical images which may be realized as a cloud storage or as a local or spread storage, e.g., as a PACS (Picture Archiving and Communication System). Further, the medical image system may comprise one or more medical imaging modalities, such as a computed tomography system, a magnetic resonance system, an angiography (or C-arm X-ray) system, a positron-emission tomography system, a mammography system, or the like.

According to other aspects, the systems are adapted to implement the inventive method in their various aspects for providing a candidate medical finding. The advantages described in connection with the method aspects may also be realized by the correspondingly configured systems' components.

According to another aspect, the present invention is directed to a computer program product comprising program elements which induce a computing unit of a system configured to provide a candidate medical finding based on a medical image to perform the steps according to one or more of the above method aspects, when the program elements are loaded into a memory of the computing unit.

According to another aspect, the present invention is directed to a computer-readable medium on which program elements are stored that are readable and executable by a computing unit of a system for providing a candidate medical finding based on a medical image according to one or more method aspects, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts an embodiment of a system for providing a candidate medical finding based on a medical image;
FIG. 2 schematically depicts a method for providing a candidate medical finding based on a medical image according to an embodiment;
FIG. 3 schematically depicts optional method steps in a method for providing a candidate medical finding based on a medical image according to an embodiment; and
FIG. 4 schematically depicts a data flow diagram in a method for providing a candidate medical finding based a medical image according to an embodiment.

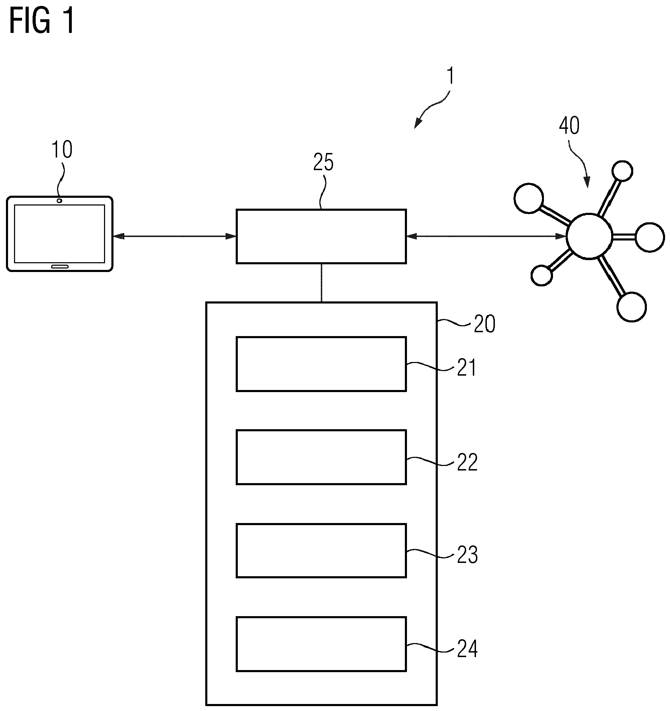

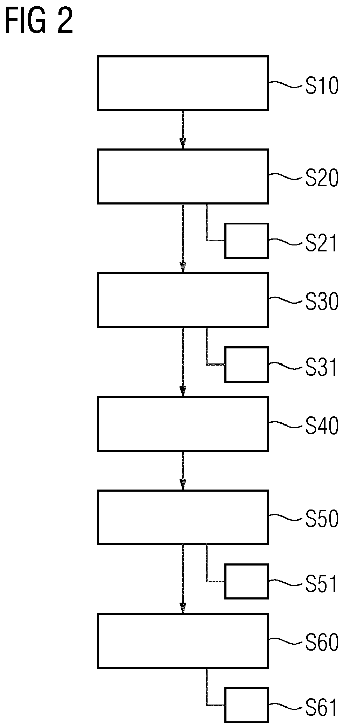

Figure 1 depicts a system 1 for providing a candidate medical finding ADD-CMF based on a medical image IM. In this regard, system 1 is adapted to perform the methods according to one or more embodiments, e.g., as further described with reference to Figures 2 to 4. A user of system 1, according to some examples, may generally relate to a healthcare professional such as a physician, clinician, technician, radiologist, pathologist and so forth.

System 1 comprises a user interface 10 (as part of the interface unit) and a processing system 20 (as part of the computing unit). Further, system 1 may comprise or be connected to a medical information system 40. The medical information system 40 may generally be configured for acquiring and/or storing and/or forwarding medical images IM. For instance, medical information system 40 may comprise one or more archive/review station (not shown) for medical images IM. The archive/review stations may be embodied by one or more databases. In particular, the archive/review stations may be realized in the form of one or more cloud storage modules. Alternatively, the archive/review stations may be realized as a local or spread storage, e.g., as a PACS (Picture Archiving and Communication System). According to some examples, medical information system 40 may also comprise one or more medical imaging modalities (not shown), such as a computed tomography system, a magnetic resonance system, an angiography (or C-arm X-ray) system, a positron-emission tomography system, a mammography system, an X-ray system, or the like.

Medical images IM may be three-dimensional image data sets acquired, for instance, using an X-ray system, a computed tomography system or a magnetic resonance imaging system or other systems. The image information may be encoded in a three-dimensional array of m times n times p voxels. Medical images IM may include a plurality of image slices which are stacked in a stacking direction to span the image volume covered by the medical images IM.

Further, medical images IM may comprise two-dimensional medical image data with the image information being encoded in an array of m times n pixels. According to some examples, these two-dimensional medical images IM may have been extracted from three-dimensional medical image data sets.

An ensemble of voxels or pixels may be designated as image data of the respective medical image IM in the following. In general, any kind of imaging modalities and scanners may be used for acquiring such image data. Generally, medical images IM show a body part or an anatomical region or an anatomic object of a patient which may comprise various anatomies and organs. Considering the chest area as a body part, medical images IM might, for instance, depict the lung lobes, the rib cage, the heart, lymph nodes, and so forth.

Medical images IM may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as the patient's name, ID, etc., and also special attributes containing the image pixel data and metadata extracted from the image data.

User interface 10 may comprise a display unit and an input unit. User interface 10 may be embodied by a mobile device such as a smartphone or tablet computer. Further, user interface 10 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen and any combination thereof. The display unit may be configured for displaying the medical images IM, receiving any user input INP, e.g., for defining a region of interest ROI, and any results and images derived therefrom in the course of the method execution such as the representations RE and RE'.

User interface 10 may further comprise an interface computing unit configured to execute at least one software component for serving the display unit and the input unit in order to provide a graphical user interface for allowing the user to select a target patient's case to be reviewed and making various inputs INP. In addition, the interface computing unit may be configured to communicate with medical information system 40 or processing system 20 for receiving the medical image IM. The user may activate the software component via user interface 10 and may acquire the software component, e.g., by downloading it from an internet application store. According to an example, the software component may also be a client-server computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. User interface 10 may also be embodied as a client.

Processing system 20 may comprise sub-units 21-24 configured to process the medical image IM in order to provide one or more candidate medical findings ADD-CMF which are indicated by the image data comprises in the medical image IM, and, optionally, to provide a representation RE of the medical image IM with candidate medical findings CMF1, ADD-CMF highlighted.

Processing system 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. The processing system 20 may be comprised in the user interface 10. Alternatively, processing system 20 may comprise a real or virtual group of computers like a so called 'cluster' or 'cloud'. Such server system may be a central server, e.g., a cloud server, or a local server, e.g., located on a hospital or radiology site. Further, processing system 20 may comprise a memory such as a RAM for temporally loading the medical image IM. According to some examples, such memory may as well be comprised in user interface 10.

Sub-unit 21 is a data retrieval module or unit. It is configured to access and search the medical information system 40 for the medical image IM. Specifically, sub-unit 21 may be configured to formulate search queries and parse them to medical information system 40.

Sub-unit 22 can be conceived as a candidate medical findings generation module or unit. It is configured to process the medical image IM in order to identify candidate medical findings in the image date of the medical image IM. Specifically, sub-unit 22 may be configured to derive two different sets of candidate medical findings CMF1 and CMF2 from the medical image IM. The two different sets of candidate medical findings CMF1 and CMF2 provide sets of candidate medical findings that are at least to some extend complementary. For instance, the first set of candidate medical findings CMF1 can be conceived as a "standard" result which has been generated based on a reasonable compromise between specificity and sensitivity. By consequence, the first set of candidate medical findings CMF1 should already comprise the majority of medical findings comprised in the medical image as candidate medical findings and at the same time a limited number of false positives, i.e., candidate medical findings that do not actually relate to "real" medical findings. The second set of candidate medical findings CMF2, by contrast, may comprise additional or complementary candidate medical findings which are not comprised in the first set of candidate medical findings CMF1. The second set of candidate medical findings CMF2 may be generated with a higher level of sensitivity SL2 than the first set of candidate medical findings CMF1. By consequence, the second set of candidate medical findings CMF2 may generally comprise more candidate medical findings than the first set of candidate medical findings CMF1 but also an increased number of false positives.

To provide the different sets of candidate medical findings, sub-unit 22 may be configured to run an accordingly configured medical findings detection algorithm ALG. In order to generate the first set of candidate medical findings CMF1, the medical findings detection algorithm ALG may be adjusted to work with a first sensitivity level SL1 by sub-unit 22. In order to generate the second set of candidate medical findings CMF2, the medical findings detection algorithm ALG may be adjusted to work with a second sensitivity level SL2 by sub-unit 22. Thereby, the second sensitivity level SL2 may be higher than the first sensitivity level SL1. According to some examples, first and second levels of sensitivity SL1, SL2 may be set by adjusting the point of operation of the medical findings detection algorithm ALG. According to other examples, different medical findings detection algorithms may be used for generating first and second sets of candidate medical findings CMF1, CMF2.

Sub-unit 23 may be configured as a user interaction module or unit. Sub-unit 23 may be configured to provide a representation RE, RE' for displaying to the user via the user interface 10. The representation RE, RE' can be in the form of a rendering in which the candidate medical findings are visually encoded. Specifically, sub-unit 23 may be configured to run or execute an algorithm for rendering a semi-transparent overlay image based on the candidate medical findings CMF1, ADD-CMF to be superimposed over the correspondingly rendered medical image IM. Thereby, sub-unit 23 may be configured to highlight candidate medical findings CMF1, ADD-CMF by indications I, I' such as symbols, boxes or labels in the medical image IM. Thereby, the results based on the "sensitive" calculation process D-PROC2, i.e., the second set of candidate medical findings CMF2, shall not be shown to the user at first per default. Only the results from the "standard" calculation process D-PROC1, i.e., the first set of candidate medical findings CMF1, shall be integrated and displayed in the initial representation RE.

Further, sub-unit 23 may be configured to define a region of interest ROI in the medical image IM based on a corresponding input INP by the user. For instance, sub-unit 23 may be configured to provide a corresponding tool, the user may activate via the user interface 10. After the tool has been activated, user inputs such as speech, gesture, eye movement, handling of input devices such as computer mouses, etc. may be evaluated to derive a region of interest ROI. According to an example, such a user input INP may designate a point or group of points in the representation RE which is then further processed to define the region of interest ROI in the medical image IM. Specifically, the point or point group may be expanded or broadened by a predetermined offset to define a region of interest ROI of a reasonable size.

Further, sub-unit 23 may be configured to also receive and process other kinds of user inputs to control the method and allow for a continued and guided human-machine interaction. Such user inputs may by directed to set first and/or second levels of sensitivity SL1, SL2, to accept or reject candidate medical findings ADD-CMF, to set the offset for defining the region of interest ROI, and/or to set a confidence interval for candidate medical findings CMF2 that are to be considered in the processing (see below).

Sub-unit 24 may be conceived as a "pull" unit configured to add medical findings from the second set of candidate medical findings CMF2 on demand. Specifically, sub-unit 24 may be configured to evaluate if the region of interest ROI comprises one or more findings of the second set of candidate medical findings CMF2 and, if so, provide these additional candidate medical findings ADD-CMF.

The designation of the distinct sub-units 21-24 is to be construed by way of example and not as a limitation. Accordingly, sub-units 21-24 may be integrated to form one single unit (e.g., in the form of "the computing unit 30") or can be embodied by computer code segments configured to execute the corresponding method steps running on a processor or the like of processing system 20. The same holds true with respect to the interface computing unit. Each sub-unit 21-24 and the interface computing unit may be individually connected to other sub-units and/or other components of the system 1 where data exchange is needed to perform the method steps. For example, sub-unit 21 may be connected via an interface 25 to medical information system 40 for retrieving the medical image IM. Likewise, interface 25 may connect the sub-units 21 to 24 to the user interface 10 for forwarding the results of the computation to the user and collect user inputs.

Processing system 20 and the interface computing unit together may constitute the computing unit of the system 1. Of note, the layout of this computing unit, i.e., the physical distribution of the interface computing unit and sub-units 21-24 is, in principle, arbitrary. For instance, sub-unit 23 (or individual elements of it or specific algorithm sequences) may likewise be localized in user interface 10. The same holds true for the other sub-units 21-24. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as a "frontend" or "client" facing the user, while processing system 20 could then be conceived as a "backend" or server. Communication between user interface 10 and processing system 20 may be carried out using the httpsprotocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Individual components of system 1 may be at least temporarily connected to each other for data transfer and/or exchange. User interface 10 communicates with processing system 20 via (data) interface 25 to exchange, e.g., medical images IM, or the result CMF1, CMF2, ADD-CMF of the computation. For example, processing system 20 may be activated on a request-base, wherein the request is sent by user interface 10. Further, processing system 20 may communicate with medical information system 40 in order to retrieve a target patient's case. As an alternative or in addition to that, user interface 10 may communicate with medical information system 40 directly. Medical information system 40 may likewise be activated on a request-base, wherein the request is sent by processing system 20 and/or user interface 10. Data interface 25 for data exchange may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Interface 25 for data exchange together with the components for interfacing with the user be regarded as constituting an interface unit of system 1.

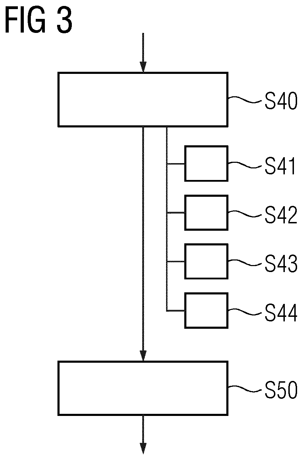

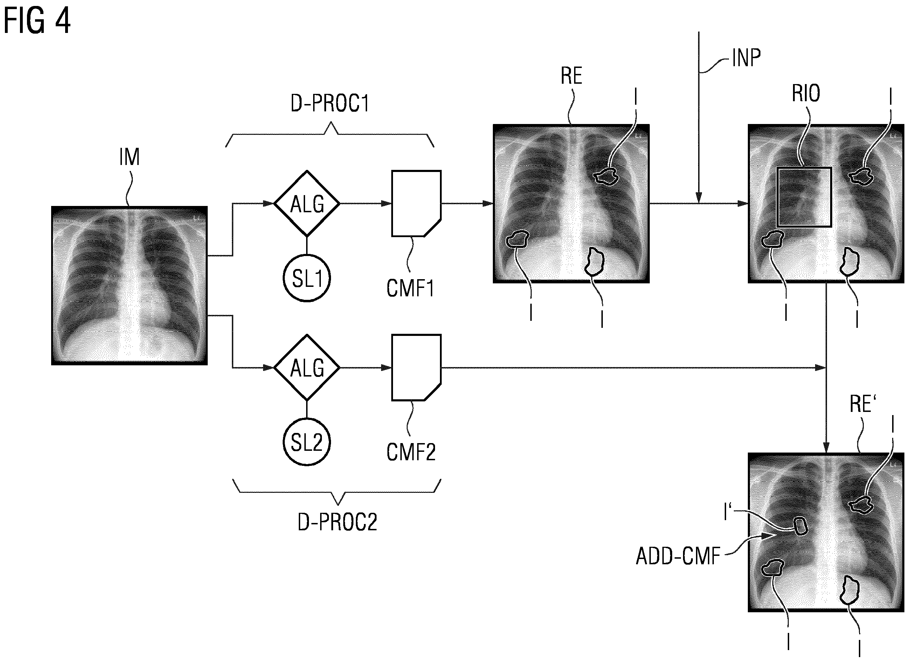

Figure 2 depicts a method for providing a candidate medical finding ADD-CMF for a medical image IM according to an embodiment. Additional optional sub-steps according to some embodiments are shown in Figure 3. Corresponding data streams are illustrated in Figure 4. The method comprises several steps. The order of the steps does not necessarily correspond to the numbering of the steps but may also vary between different embodiments of the present invention. Further, individual steps or a sequence of steps may be repeated.

In a first step S10, the medical image IM is received. This may involve selecting the medical image IM from a plurality of cases, e.g., stored in the medical information system 40. The selection may be performed manually by a user, e.g., by selecting appropriate image data in a graphical user interface running in the user interface 10. Alternatively, the medical image IM may be provided to the computing unit by a user by way of uploading the medical image IM to the computing unit.

In a next step S20, a first image processing step D-PROC1 is performed on the medical image IM. The first image processing step D-PROC1 is a medical findings detection process directed to detect a first set of candidate medical findings CMF1. The first medical findings detection process D-PROC1 detects candidate medical findings with a first sensitivity level SL1. Detecting the first set of medical findings CMF1 may involve applying a medical findings detection algorithm ALG on the medical image IM which algorithm ALG has been set to operate at the first sensitivity level SL1. This may be done by appropriately setting the point of operation of the medical findings detection algorithm ALG.

An optional sub-step S21 of step S20 is directed to set the first sensitivity level SL1. Basically, the first sensitivity level SL1 may be set manually by a user (by making an appropriate user input in the user interface 10), automatically by the system 1 or semi-automatically. An automatic setting may involve evaluating the medical image IM and any supplementary information available for the medical image IM to derive a suitable value for the first sensitivity level SL1. For instance, this may involve determining the diagnostic circumstances (which kinds of medical findings are to be detected), the image quality, and/or known user preferences of the user.

The first sensitivity level SL1 may generally be adjusted such that a good compromise between sensitivity and specificity of the detection is achieved. With that, a first sensitivity level SL1 can be provided that works well for most findings in the medical image IM. A semi-automatic setting may mean automatically providing suggestions to the user as to an appropriate first sensitivity level SL1 and setting the first sensitivity level SL1 based on a user selection of one of the suggestions.

At step S30, a second medical findings detection process D-PROC2 is carried out. The second medical findings detection process D-PROC2 is directed to provide a second set of candidate medical findings CMF2 based on the medical image IM. The second medical findings detection process D-PROC2 detects candidate medical findings with a second sensitivity level SL2. Detecting the second set of medical findings CMF2 may likewise involve applying a medical findings detection algorithm ALG on the medical image IM. Thereby, the algorithm used in the second medical findings detection process D-PROC2 may be identical to the one used at the first medical findings detection process D-PROC1. However, in the second medical findings detection process D-PROC2, the medical findings detection algorithm ALG is set to operate at the second sensitivity level SL2. This may be done by appropriately setting the point of operation of the medical findings detection algorithm ALG. The second sensitivity level SL2 may be higher than the first sensitivity level SL1. Step S20 and step S30, that is the two medical findings detection processes D-PROC1 and D-PROC2, may run in parallel.

An optional sub-step S31 of step S30 is directed to set the second sensitivity level SL2. Like the first sensitivity level SL1, the second sensitivity level SL2 may be set manually by the user, automatically, or semi-automatically essentially as described before in connection with step S21. Additionally, the second sensitivity level SL2 may be set based on the first sensitivity level SL1 so as to optimally complement the first sensitivity level SL1. For instance, the second sensitivity level SL2 may be set such that there is a reasonable difference in sensitivity between the first and second medical findings detection process D-PROC1, D-PROC2.

At step S40, a region of interest ROI is obtained which is to be analyzed for candidate medical findings ADD-CMF comprised in the second set of medical findings CMF2 which could complement the candidate medical findings of the first set of candidate medical findings CMF1. An example of a corresponding human-machine interaction will be illustrated by way of the optional method steps of Figure 3.

At step S41, a representation RE of the medical image IM for display to the user via the user interface 10 is rendered. As shown in Figure 4, the representation RE comprises indications I of the candidate medical findings of the first set of candidate medical findings CMF1. The indications I may indicate the position and/or outline of the candidate medical findings in the medical image IM. The representation RE does not comprise any indication of candidate medical findings of the second set of medical findings CMF2 as their lower degree of specificity (and potentially higher number of false positives) could potentially overwhelm the user.

At step S42, the representation RE is provided to the user. That followed, at step S43, a user input INP is received directed to define a region of interest ROI in the representation RE and, therewith, in the medical image IM. As mentioned, the user input INP may be based on various kinds of user interactions with the system 1, such as inputs via the aforementioned input units or devices including but not limited to speech inputs, inputs generated by eye tracking, gesture commands, direct inputs using input devices etc. The user input INP may be such that it fully defines the region of interest ROI. For instance, a user may directly input the entire outline of the intended region of interest ROI. According to alternative examples, the user input INP may define an appropriate region of interests ROI only partially by specifying only one or more points or pixels in the representation RE.

At step S44, the region of interest ROI is defined based on the user input INP. According to some embodiments, this may involve complementing or extending the user input INP where needed. Thereby the human machine interaction is rendered more comfortable, and the user is provided with additional assistance. In particular, if the user input INP only designates a point or pixel, the region of interest ROI may be, preferably circularly, extended by an offset around the point or pixel designated by the user. The offset may be measured in pixels. Further, the offset may be set by the user, e.g., upon activating the tool with which the region of interest ROI is defined for searching for additional candidate medical findings from the second set CMF2. The region of interest ROI may or may not be visualized in the representation RE.

At step S50, at least one candidate medical finding ADD-CMF is identified within the region of interest ROI which is not yet comprised in the first set of candidate medical findings CMF1. Specifically, it may be determined in optional sub-step S51 if the region of interest ROI comprises any candidate medical findings of the second set of medical findings CMF2. To this end, the second set of candidate medical findings CMF2 may be searched for candidate medical findings located in the region of interest ROI. If the second set of candidate medical findings CMF2 comprises such candidate medical findings it may optionally be checked whether or not they are already comprised in the first set of medical findings CMF1 to avoid duplications. The additional candidate medical findings ADD-CMF thus detected may then be provided in subsequent step S60. If the region of interest ROI does not comprise any candidate medical findings of the second set of candidate medical findings CMF2, this may likewise be brought to the attention to the user, e.g., with an appropriate notification to the user via the user interface 10.

At step S60, the additional candidate medical findings ADD-CMF identified at step S50 are provided. Providing may mean that the additional candidate medical findings ADD-CMF stemming from the set of second candidate medical findings CMF2 are appended to the first set of candidate medical findings CMF1. This may mean that the additional candidate medical findings ADD-CMF are stored together with the candidate medical findings of the first set CMF1. This may also mean that the additional candidate medical findings ADD-CMF are associated to the first set CMF1 such that they can be retrieved in conjunction with the candidate medical findings of the first set CMF1. Further, step S60 may comprise including the additional candidate medical findings ADD-CMF in a medical report.

Further, providing may mean that the additional candidate medical finding ADD-CMF is included in the representation RE alongside the candidate medical findings of the first set CMF1. This may happen in optional sub-step S61. Specifically, a modified representation RE' may be generated similarly as in step S41. This modified representation RE' may comprise additional indications I' directed to the additional candidate medical findings ADD-CMF pulled from the second set CMF2 (c.f., Figure 4).

According to an embodiment, step S60 may comprise a confirmation interaction with the user. Here, the user may evaluate whether or not the additional candidate medical findings ADD-CMF are sound and are to be included in the first set CMF1, for instance. To this end, the user interface 10 may be configured to receive a user input direct to decline or accept the additional candidate medical finding ADD-CMF and process the additional candidate medical finding ADD-CMF accordingly.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention.

## Claims

1. Computer-implemented method for providing a candidate medical finding comprised in a medical image, the method comprising the following steps:
- obtaining (S10) the medical image (IM) depicting a body part of a patient,
- generating (S20) a first set of candidate medical findings (CMF1) by subjecting the medical image (IM) to a first medical findings detection process (D-PROC1),
- generating (S30) a second set of candidate medical findings (CMF2) by subjecting the medical image (IM) to a second medical findings detection process (D-PROC2) different than the first medical findings detection process (D-PROC1),
- obtaining (S40) a region of interest (ROI) in the medical image (IM),
- identifying (S50), in the region of interest (ROI), at least one candidate medical finding (ADD-CMF) comprised in the second set of candidate medical findings (CMF2) and not comprised in the first set of candidate medical findings (CMF1), and
- providing (S60) the at least one candidate medical finding (ADD-CMF).

2. Method according to claim 1, wherein
- the step of generating (S20) the first set of candidate medical findings (CMF1) comprises detecting candidate medical findings in the medical image (IM) with a first sensitivity level (SL1), and
- the step of generating (S30) the second set of candidate medical findings (CMF2) comprises detecting candidate medical findings in the medical image (IM) with a second sensitivity level (SL2) higher than the first sensitivity level (SL1).

3. Method according to claim 2, wherein
- in the step of generating (S20) the first set of candidate medical findings (CMF1), the first medical findings detection process (D-PROC1) comprises applying a first medical findings detection algorithm (ALG) to the medical image (IM), the first medical findings detection algorithm (ALG) operating at the first sensitivity level (SL1), and
- in the step of generating (S30) the second set of candidate medical findings (CMF2), the second medical findings detection process (D-PROC2) comprises applying a second medical findings detection algorithm (ALG2) to the medical image (IM), the second medical findings detection algorithm (ALG2) operating at the second sensitivity level (SL2).

4. Method according to claim 3, wherein
- the second medical findings detection algorithm (ALG) and the first medical image algorithm (ALG) are the same.

5. Method according to any one of claims 2 to 4, further with the step of
- setting (S21) the first sensitivity level (SL1) based on any one of:
- an input of the user directed to set the first sensitivity level (SL1),
- the medical image (IM), and/or
- supplementary non-image data associated with the medical image (IM).

6. Method according to any one of claims 2 to 5, further with the step of
- setting (S31) the second sensitivity level (SL2) based on the first sensitivity level (SL1).

7. Method according to any of the preceding claims, wherein
- the first medical findings detection process (D-PROC1) and the second medical findings detection process (D-PROC1) run in parallel.

8. Method according to any one of the preceding claims, wherein
- the step of identifying (S50) the at least one candidate medical finding (ADD-CMF) comprises determining (S51) if the region of interest (ROI) comprises the at least one candidate medical finding (ADD-CMF), and
- the step of providing (S60) the at least one candidate medical finding (ADD-CMF) is based on the step of determining (S50) .

9. Method according to any one of the preceding claims, wherein the step of obtaining (S40) the region of interest (ROI) comprises:
- generating (S41) a representation (RE) of the medical image (IM) for displaying to a user in a user interface (10), wherein, optionally, the representation (RE) comprises one or more indications (I) of the candidate medical findings (CMF1) comprised in the first set of candidate medical findings (CMF1) ,
- providing (S42) the representation (RE) to the user in the user interface (10),
- receiving (S43), optionally via the user interface (10), a user input (INP) directed to indicate the region of interest (ROI) in the representation (RE), and
- defining (S44) the region of interest (ROI) in the medical image (IM) based on the user input (INP) directed to indicate the region of interest (ROI).

10. Method according to claim 9, wherein
- the user input (INP) is directed to a location in the representation (RE), and
- the region of interest (ROI) is defined as a region around the location within a preset distance from the location.

11. Method according to any one of claims 9 or 10, wherein the step of providing (S60) comprises
- including (S61) an indication (I') of the at least one candidate medical finding (ADD-CMF) in the representation (RE) so as to generate an updated representation (RE'), further with the step of
- displaying the updated representation (RE') to the user in the user interface (10).

12. Method according to any of the preceding claims, wherein
each of the candidate medical findings (CMF2) of the second set of candidate medical findings (CMF2) comprises a confidence value, and
in the step of identifying (S40) the at least one candidate medical finding (ADD-CMF) only such candidate medical findings (CMF2) of the second set of medical findings (CMF2) are considered the confidence values of which are above a preset confidence threshold.

13. System (1) for providing a candidate medical finding (ADD-CMF) comprised in a medical image (IM), the system 81= comprising an interface unit (10, 25) and a computing unit (20), wherein
- the interface unit (10, 25) is configured to obtain (S10) the medical image (IM) depicting a body part of a patient, and
- the computing unit (20) is configured to:
- generate (S20) a first set of candidate medical findings (CMF1) by subjecting the medical image (IM) to a first medical findings detection process (D-PROC1),
- generate (S30) a second set of candidate medical findings (CMF2) by subjecting the medical image (IM) to a second medical findings detection process (D-PROC2) different than the first medical findings detection process (D-PROC1),
- obtain (S40) a region of interest (ROI) in the medical image (IM),
- identify (S50), in the region of interest (ROI), at least one candidate medical finding (ADD-CMF) comprised in the second set of candidate medical findings (CMF2) and not comprised in the first set of candidate medical findings (CMF1), and
- provide (S60) the at least one candidate medical finding (ADD-CMF).

14. Computer program product comprising program elements which induce a computing unit (20) of a system (1) for providing a candidate medical finding (ADD-CMF) for a body part of a patient based on a medical image (IM) of the body part of the patient to perform steps of the method according to any of claims 1 to 12 when the program elements are loaded into a memory of the computing unit (20).

15. Computer-readable medium on which program elements are stored that are readable and executable by a computing unit (20) of a system (1) for providing a candidate medical finding for a body part of a patient based on a medical image (IM) of the body part of the patient to perform steps of the method according to any of claims 1 to 12, when the program elements are executed by the computing unit (20).
